# EUROPEAN PATENT APPLICATION

(11) **EP 4 607 432 A1**
(43) Date of publication of application: **27.08.2025**
(21) Application number: 24747356.4
(22) Date of filing: 25.01.2024
(51) Int. Cl.: G06Q 30/0202, A61B 5/16

(54) **EVALUATION VALUE ESTIMATION SYSTEM, EVALUATION VALUE ESTIMATION METHOD, EVALUATION VALUE ESTIMATION DEVICE, EVALUATION VALUE ESTIMATION PROGRAM, AND STORAGE MEDIUM ON WHICH SAID PROGRAM IS RECORDED**

(30) Priority: 25.01.2023 JP 2023009521; 26.09.2023 JP 2023163665
(71) Applicant: Kikkoman Corporation, Noda-shi, Chiba 278-8601 (JP)
(72) Inventor: HIRAGUCHI, Haruka, 6709 PA Wageningen (NL); SATO, Takuya, 6709 PA Wageningen (NL)
(74) Representative: Schön, Christoph
(86) International application number: PCT/JP2024/002236
(87) International publication number: WO 2024/158032

(57) **Abstract**

An estimation system S includes: an affect recognition unit 12 that recognizes valence intensity and arousal of a user toward a subject thing; and an evaluation value estimation unit 16 that estimates a user evaluation value by using a learning model that receives the valence intensity and the arousal of the user as an input and outputs the user evaluation value.

## Description

### Technical Field

The present invention relates to an evaluation value estimation system, an evaluation value estimation method, an evaluation value estimation device, and an evaluation value estimation program that estimate an aspiration of a user for a subject thing, as well as a storage medium on which the program is recorded.

### Background Art

Generally, the contents and intensities of affect vary from person to person even if the affect is experienced toward the same thing. Moreover, it is difficult to express an affect accurately and quantitatively, even though the affect is of one's own. Further, since affect toward a certain thing changes over time, it is desirable that an affect be expressed immediately. On the other hand, a need exists to quantitatively understand respective affects of many people toward a certain thing in a short time.

To meet such a need, various methods for measuring affects have been proposed. For example, in a technology described in Patent Literature 1, a human body-like model having joints is used, and affects experienced by a user are identified based on how and which j oint or joints the user bends.

### Citation List

### Patent Literature

Patent Literature 1: Japanese Patent No. 6508988

### Summary of Invention

### Technical Problem

However, when a user is made to express affects by using a model as in the technology described in Patent Literature 1, a certain explanation is needed about a method for expressing the affects. Moreover, even if expressions represented by the model are similar, affects associated with the expressions may vary from user to user, depending on the cultural background or the like. Further, for a user to express an affect by using a model, a certain period of time is required to operate the model, and a change may occur in the affect felt at the time of contact with a thing.

Accordingly, there has been a possibility that data related to affects collected by using such a model is of less versatility. For example, when analysis is performed based on data collected from users inhabiting a certain region, a result of the analysis is not always applicable to users inhabiting another region.

Moreover, when a model with such a complicated structure is used, the problem also arises that entry work for a user to do is complicated, so that it is difficult to collect data from many users.

The present invention has been made in view of the above respects, and an object thereof is to provide an evaluation value estimation system, an evaluation value estimation method, an evaluation value estimation device, and an evaluation value estimation program that make it possible to quickly and easily collect data related to affects of users in a highly versatile state, and further to easily analyze the collected data, as well as a storage medium on which the program is recorded.

### Solution to Problem

An evaluation value estimation system of the present invention is an evaluation value estimation system that estimates a user evaluation value that is a value concerning aspiration of a user for a subject thing, and includes: an affect recognition unit that recognizes valence intensity and arousal of the user toward the subject thing; and an evaluation value estimation unit that estimates the user evaluation value by using a learning model that receives the valence intensity and the arousal of the user as an input and outputs the user evaluation value.

Here, "valence" is a bipolar concept that indicates qualitative differences in affect evoked (for example, a positive value such as attractiveness (pleasant) of, or a negative value such as averseness (unpleasant) to, a subject thing). The "valence intensity" is the intensity of how strongly an affect of interest is felt (Harabe, Yoshihiro (2015). Measurement of general emotional valence intensity by Thurstone's method of paired comparisons. - Preliminary study: using unpleasant word - Annual report (17), Faculty of Human Sciences, Tezukayama Gakuin University).

The "arousal" is a concept that has excited (high arousal) and calm (low arousal) at polar ends, and indicates the intensity of an affect evoked (Bradley, M.M., Greenwald, M.K., Petry, M.C., and Lang, P.J. (1992). Remembering Pictures: Pleasure and Arousal in Memory. Journal of Experimental Psychology: Learning, Memory, and Cognition, 18, 379-390.).

An "evaluation value" is a value concerning aspiration for a subject thing or a similar thing. For the evaluation value, adoptable is what can be expressed by distinct values by using a point scale or the like with any values set thereon, such as the frequency of past consumption, the degree of recommendation to others, the degree of consuming intention, or the degree of buying intention.

As described above, the evaluation value estimation system of the present invention adopts universal parameters, valence and arousal. Accordingly, a user evaluation value outputted as a result of such parameters being inputted is also a versatile value that is not biased by cultural background or the like. In addition, valence and arousal are parameters that can be easily acquired without using special equipment.

Furthermore, since the parameters are simplified parameters, for parameters that express complex affect, analysis processing can be performed easily and quantitatively when analysis is performed by using the parameters, compared to when a conventional parameter that expresses an affect (for example, text created by a user, or the like) is used.

Accordingly, with the system, it is possible to quickly and easily collect data related to affects of users in a versatile state, and further to easily analyze the collected data. Such advantageous effects are particularly great when a thing to be evaluated, like a dish, involves complex factors, such as cultural background or the like in addition to personal physical condition and preference.

In the evaluation value estimation system of the present invention, preferably, the affect recognition unit includes: an affect model presentation section that presents, to the user, an affect model that is a two-dimensional model or a three-dimensional model including at least a first axis representing valence intensity and a second axis representing arousal, in a form in which any coordinates in the affect model are selectable; and a selected-coordinate recognition section that recognizes selected coordinates that are coordinates selected by the user from the affect model when the subject thing is presented, and the evaluation value estimation unit receives the selected coordinates as an input.

When an affect model that is a two-dimensional model or a three-dimensional model including at least a first axis representing valence intensity and a second axis representing arousal is adopted as an interface to which an affect is inputted as described above, a user can easily and accurately express his/her own affect as a distinct value in the form of coordinates. As a result, it is possible to collect data related to affects of users easily and accurately.

Moreover, since the parameters in the form of coordinates are parameters that can be intuitively understood by a user, it is not necessary to give users a particular explanation about a method for expression when collecting data. Thus, it is possible to quickly collect parameter values related to an affect that can easily change before the affect changes. Since the parameters in the form of coordinates are simple and distinct, analysis processing can be performed more easily if such coordinates are used for the parameters to perform analysis.

Note that for a third axis in a case where the affect model is a three-dimensional model, for example, an axis representing the degree of extent of arousal may be adopted (Sato, Toshio, Horike, Kazuya (2018). Study of emotional scale by 3-dimensional model, The 82nd Annual Convention of the Japanese Psychological Association, 721).

In the evaluation value estimation system of the present invention, preferably, the user evaluation value includes values concerning a plurality of evaluation items, and the learning model receives one set of values of the valence intensity and the arousal as an input and outputs the values concerning the plurality of evaluation items.

Here, the "evaluation items" are, for example, items based on five human senses in response to the subject thing. For example, when the subject thing is a dish, the taste of the dish (sense of taste), the aroma and flavor thereof (sense of smell), the look thereof (sense of vision), the texture thereof (sense of touch), the sound produced when the dish is eaten or cooked (sense of hearing), and the like are evaluation items.

The user evaluation value does not necessarily need to be a value concerning one evaluation item, and may include values concerning a plurality of evaluation items as described above. When a plurality of evaluation items are adopted for the user evaluation value as described above, since multifaceted evaluations can be performed, it is possible to make a result of analysis using such user evaluation values more objective and accurate.

When the evaluation value includes the values concerning the plurality of evaluation items, the evaluation value estimation system of the present invention, preferably, includes an attractiveness value estimation unit that estimates, as an attractiveness value, a value obtained by weighting each of the values concerning the plurality of evaluation items outputted from the learning model as a result of the one set of values of the valence intensity and the arousal being inputted, and adding up the weighted values.

There are some cases where an evaluation item to be weighted more heavily may vary, depending on a subject thing of which evaluation values are to be estimated, the purpose of use of user evaluation values, or the like. Accordingly, when an attractiveness value that is a single value is outputted by adding up user evaluation values as described above, calculation of the attractiveness value is configured to be performed after attaching of weights that vary from evaluation item to evaluation item is performed in a manner according to the purpose of use or the like. Thus, the outputted attractiveness value, and hence a result of analysis using the attractiveness value, can be made more accurate.

In the evaluation value estimation system of the present invention, when the user evaluation value includes the values concerning the plurality of evaluation items, preferably, the subject thing is food, and the plurality of evaluation items includes at least one of an evaluation item based on a sense of taste, an evaluation item based on a sense of smell, an evaluation item based on a sense of vision, and an evaluation item based on a sense of touch.

In general, since evaluation of food is performed with regard to a plurality of items, such as taste (sense of taste), aroma and flavor (sense of smell), a look (sense of vision), texture (sense of touch), and sound produced when the food is eaten or cooked (sense of hearing), outputted evaluations can be easily complicated. Accordingly, for such evaluation of food, when the system is used that easily and quickly collects data related to affects as input data, and that outputs a user evaluation value that is easy to handle at a time of analysis, it is possible to perform the evaluation easily and accurately.

In the evaluation value estimation system of the present invention, preferably, the user evaluation value includes values concerning a plurality of evaluation metrics, and the learning model receives one set of values of the valence intensity and the arousal as an input and outputs the values based on the plurality of evaluation metrics.

Here, the "evaluation metrics" are metrics for evaluation in terms of aspiration for the subject thing or a similar thing. Accordingly, "evaluation values" in such a case are values in the evaluation metrics. Note that for the evaluation metrics, for example, the frequency of past consumption, the degree of recommendation to others, the degree of consuming intention, the degree of buying intention, and the like are adoptable.

The user evaluation value does not necessarily need to be one value, and may include values concerning a plurality of evaluation metrics (that is, a plurality of values) as described above. When values concerning a plurality of evaluation metrics are adopted as described above, since multifaceted evaluations can also be performed on one item, it is possible to make a result of analysis using such user evaluation values more objective and accurate.

When the user evaluation value includes the values concerning the plurality of evaluation metrics, the evaluation value estimation system of the present invention, preferably, includes an attractiveness value estimation unit that recognizes, as an attractiveness value, a value obtained by weighting each of the values concerning the plurality of evaluation metrics outputted from the learning model as a result of the one set of values of the valence intensity and the arousal being inputted, and adding up the weighted values.

There are some cases where an evaluation metric to be weighted more heavily may vary, depending on a subject thing of which user evaluation values are to be estimated, the purpose of use of user evaluation values, or the like. Accordingly, when an attractiveness value that is a single value is outputted by adding up user evaluation values as described above, calculation of the attractiveness value is configured to be performed after attaching of weights that vary from evaluation metric to evaluation metric is performed in a manner according to the purpose of use or the like. Thus, the outputted attractiveness value, and hence a result of analysis using the attractiveness value, can be made more accurate.

In the evaluation value estimation system of the present invention, when the evaluation value includes the values concerning the plurality of evaluation metrics, the values concerning the plurality of evaluation metrics may include at least one of a value concerning frequency of past consumption, a value concerning a degree of recommendation to others, a value concerning a degree of consuming intention, and a value concerning a degree of buying intention.

In the evaluation value estimation system of the present invention, when the affect model that is a two-dimensional model or a three-dimensional model including at least a first axis representing valence intensity and a second axis representing arousal is adopted as an interface for receiving an affect as an input, the learning model may be correlation data indicating a correlation between reference coordinates and a reference evaluation value, the reference coordinates being coordinates selected from the affect model, the reference evaluation value being a value concerning aspiration for the subject thing or a similar thing having an identical or similar attribute to an attribute of the subject thing.

In the evaluation value estimation system of the present invention, when the correlation data is used for the learning model, the reference coordinates may be coordinates selected with regard to the subject thing or the similar thing from the affect model by a test user who evaluates the subject thing or the similar thing beforehand, and the correlation data may be data indicating a correlation between the reference coordinates and the reference evaluation value given by the test user.

In the evaluation value estimation system of the present invention, when the affect model that is a two-dimensional model or a three-dimensional model including at least a first axis representing valence intensity and a second axis representing arousal is adopted as an interface for receiving an affect as an input, the learning model may be a prediction model that is generated through machine learning using reference coordinates and a reference evaluation value for training data, and that outputs the user evaluation value from the selected coordinates inputted, the reference coordinates being coordinates selected from the affect model, the reference evaluation value being a value concerning aspiration for the subject thing or a similar thing having an identical or similar attribute to an attribute of the subject thing.

In the evaluation value estimation system of the present invention, when the prediction model is used for the learning model, the reference coordinates may be coordinates selected with regard to the subject thing or the similar thing from the affect model by a test user who evaluates the subject thing or the similar thing beforehand, and the prediction model may be generated through machine learning using the reference coordinates and the reference evaluation value given by the test user for training data.

An evaluation value estimation device of the present invention is an evaluation value estimation device that estimates a user evaluation value that is a value concerning aspiration of a user for a subject thing, and includes: an affect recognition unit that recognizes valence intensity and arousal of the user toward the subject thing; and an evaluation value estimation unit that estimates the user evaluation value by using a learning model that receives the valence intensity and the arousal of the user as an input and outputs the user evaluation value.

An evaluation value estimation method of the present invention is an evaluation value estimation method of estimating a user evaluation value that is a value concerning aspiration of a user for a subject thing, and includes: by an affect recognition unit, recognizing valence intensity and arousal of the user toward the subject thing; and by an evaluation value estimation unit, estimating the user evaluation value by using a learning model that receives the valence intensity and the arousal of the user as an input and outputs the user evaluation value.

An evaluation value estimation program of the present invention is an evaluation value estimation program that causes a computer to execute an evaluation value estimation method of estimating a user evaluation value that is a value concerning aspiration of a user for a subject thing, and causes the computer to execute: a step of, by an affect recognition unit, recognizing valence intensity and arousal of the user toward the subject thing; and a step of, by an evaluation value estimation unit, estimating the user evaluation value by using a learning model that receives the valence intensity and the arousal of the user as an input and outputs the user evaluation value.

A recording medium of the present invention is a recording medium on which the above-described evaluation value estimation program is recorded, and from which the evaluation value estimation program is readable by the computer.

### Brief Description of Drawings

FIG. 1 is an explanatory diagram showing a schematic configuration of an estimation system according to an embodiment.
FIG. 2 is a block diagram showing a configuration of processing units of the estimation system in FIG. 1.
FIG. 3 is an image diagram showing an example of an image displayed on a user terminal when the estimation system in FIG. 1 recognizes information on a user.
FIG. 4 is an image diagram showing an example of an image displayed on the user terminal when the estimation system in FIG. 1 recognizes an affect of the user.
FIG. 5A is a schematic diagram showing an example of an affect model used for the estimation system in FIG. 1 to recognize an affect.
FIG. 5B is a schematic diagram showing an example of an affect model used for an estimation system according to a modification example to recognize an affect.
FIG. 6 is an image diagram showing an example of an image displayed on the user terminal when the estimation system in FIG. 1 recognizes reference evaluation values.
FIG. 7 is an image diagram showing an example of correlation data recognized by the estimation system in FIG. 1.
FIG. 8 is a flowchart showing a process that is executed when the estimation system in FIG. 1 collects correlation data.
FIG. 9 is a flowchart showing a process that is executed when the estimation system in FIG. 1 estimates an attractiveness value.
FIG. 10 is an image diagram showing an example of an image displayed on a client terminal after the estimation system in FIG. 1 estimates the attractiveness value.
FIG. 11 shows images of samples used in an experiment for collecting data for generating a learning model in the estimation system in FIG. 1.
FIG. 12 shows data related to the degree of buying intention (Purchase Probability Score), of the experimental data for generating the learning model in the estimation system in FIG. 1.
FIG. 13 shows data related to the degree of consuming intention (Willingness to take home), of the experimental data for generating the learning model in the estimation system in FIG. 1.
FIG. 14 is data related to the degree of recommendation to others (Net Promoter Score), of the experimental data for generating the learning model in the estimation system in FIG. 1.

### Description of Embodiments

Hereinafter, an evaluation value estimation system (hereinafter, referred to as "estimation system S") according to an embodiment and an evaluation value estimation method performed using the system are described with reference to the drawings.

In the present embodiment, a description is given of a case where the estimation system S is used for a service of estimating an attractiveness value given by a user U to a subject thing (a dish in the present embodiment), and presenting the attractiveness value to a client engaged in marketing or the like.

Note that in the description below, an "evaluation value" is a value concerning aspiration for the subject thing or a similar thing, which will be described later. Specifically, adoptable is what is expressed by distinct values by using a point scale or the like with any values set thereon, such as frequency of past consumption (for example, Frequency of past consumption or the like), the degree of recommendation to others (for example, Net promoter score or the like), the degree of consuming intention (for example, Willingness to take home or the like), the degree of buying intention (for example, Purchase Probability Score, Willingness to buy, the Juster scale, or the like), or the like. Values of those listed above are adopted in the estimation system S.

A "reference evaluation value" is an evaluation value used to generate correlation data (learning model), which will be described later, and is a value concerning aspiration of a user U as a test user for the subject thing or a similar thing.

In contrast, a "user evaluation value" is an evaluation value estimated by an evaluation value estimation unit 16, which will be described later, and is a value concerning aspiration, for the subject thing, of a user U as a person for which evaluation estimation by the estimation system S is performed (that is, a target audience for marketing).

Here, a "subj ect thing" refers to a thing to be evaluated (in other words, of which a user evaluation value is to be estimated). A "similar thing" refers to a thing having an identical or similar attribute to an attribute of the subject thing.

Note that in the present embodiment, the subject thing is assumed to be a dish. Accordingly, for similar things, dishes and the like as follows apply, such as a dish having at least one of the same taste, aroma and flavor, look, texture, and the like, or having a similar taste, as the dish that is the subject thing, a flavoring having the same, or similar, aroma and flavor as the dish, a picture of the dish or a similar dish, a dish having the same, or similar, texture as the dish, and a dish producing the same, or similar, sound as the dish when eaten or cooked. For attributes of the subject thing or a similar thing in the present embodiment, taste, aroma and flavor, a look, texture, and the like are adoptable.

Incidentally, in general, since evaluation of a dish (hence food) is performed with regard to a plurality of items, such as taste (sense of taste), aroma and flavor (sense of smell), a look (sense of vision), texture (sense of touch), and sound produced when the dish is eaten or cooked (sense of hearing), outputted evaluations can be easily complicated. Use of the evaluation value estimation system and the like of the present invention makes it possible to easily and accurately evaluate even such a thing of which evaluation can be easily complicated.

However, the evaluation value estimation system and the like of the present invention are not limited to the configurations as described above, and estimated evaluation values are not necessarily applicable only to estimation of an attractiveness value based on user evaluation values given by a user to a dish.

Accordingly, for example, the evaluation value estimation system and the like of the present invention may be used for new product marketing and the like, by estimating user evaluation values that would be given by a large number of users to a new product of food, or of anything other than food (for example, clothing or the like), and referring to the user evaluation values.

### [Schematic configuration of system]

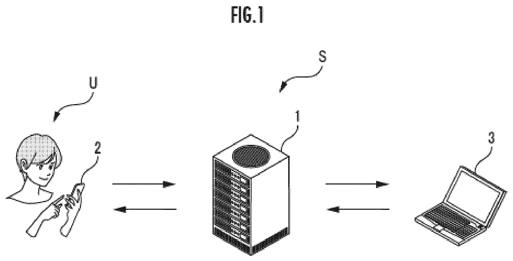

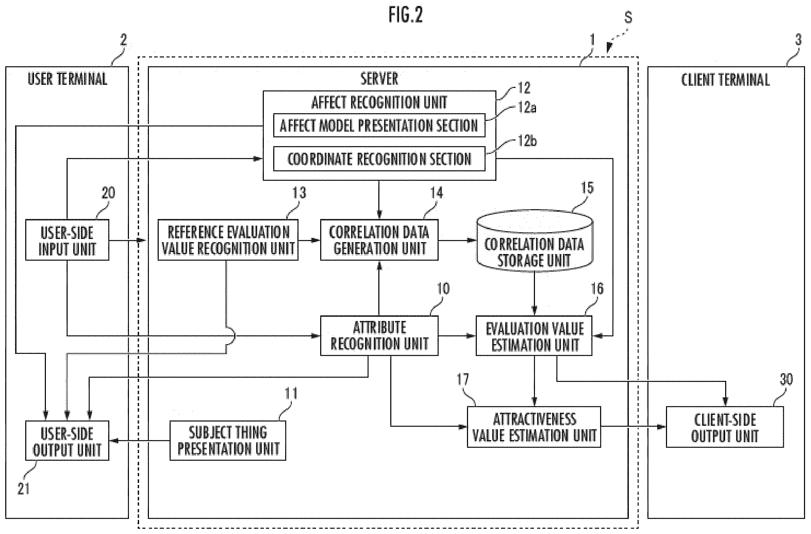

Hereinafter, with reference to FIGS. 1 and 2, a schematic configuration of the estimation system S is described.

As shown in FIG. 1, the estimation system S is a computer system and includes a server 1 held by a provider of the service that is rendered by the estimation system S.

The server 1 is configured to be able to mutually communicate information with a user terminal 2, such as a smartphone or a tablet used by a user U, and with a client terminal 3, such as a personal computer used by a client, via an Internet network, a public circuit, or the like.

Note that the evaluation value estimation system of the present invention is not limited to a configuration made by using only one server, and may be configured in such a manner that any terminal included in the evaluation value estimation system includes later-described processing units.

Accordingly, for example, the evaluation value estimation system may include a plurality of servers. For example, at least one of the user terminal and the client terminal may be equipped with at least one of the processing units, or at least part of the functionality of the processing units, and the evaluation value estimation system may be configured through cooperation between the at least one of the user terminal and the client terminal and the server, or by using the at least one of the user terminal and the client terminal.

For example, a terminal including a processing unit may be configured as an independent evaluation value estimation device by being provided with functionality corresponding to a user-side input unit 20 and a user-side output unit 21 (see FIG. 2) that are provided to the user terminal 2 in the present embodiment. Further, such an evaluation value estimation device may be provided with functionality corresponding to a client terminal-side input unit (not shown) and a client-side output unit 30 (see FIG. 2) that are provided to the client terminal 3.

As shown in FIG. 2, the user terminal 2 that can communicate with the server 1 as the estimation system S includes the user-side input unit 20 and the user-side output unit 21 as functions (processing units) implemented by at least one of a mounted hardware configuration and a program.

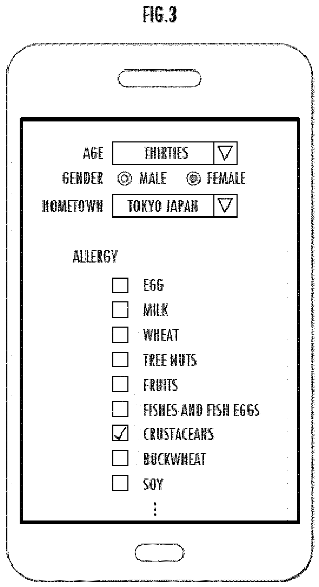

In the present embodiment, a description is given, assuming that a touch panel (see FIG. 3 and others) serves as the user-side input unit 20 and the user-side output unit 21 of the user terminal 2.

However, the user terminal is not limited to such a configuration, and may be any one that can receive information from a user as an input and can make an output for presenting information to the user. Accordingly, the user terminal may be configured to be able to receive an input and make an output by using, for example, a keyboard, a microphone, a camera, a speaker, or the like, apart from the touch panel.

The client terminal 3 that can communicate with the server 1 as the estimation system S includes the client-side input unit (not shown) and the client-side output unit 30 as functions (processing units) implemented by at least one of a mounted hardware configuration and a program.

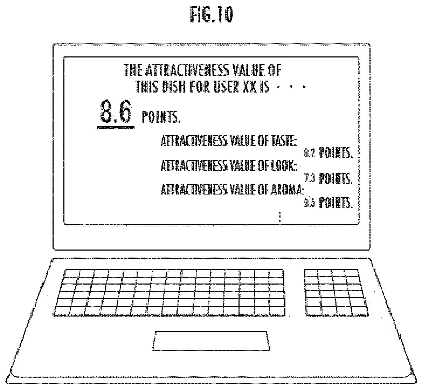

In the present embodiment, a description is given, assuming that a keyboard and a touch pad serve as the client-side input unit of the client terminal 3, and a monitor (see FIG. 10) serves as the client-side output unit 30.

However, the client terminal is not limited to such a configuration, and may be any one that can make an output for presenting information to a client. Accordingly, the client terminal may be configured to be able to make an output by using, for example, a speaker or the like.

Note that the user terminal and the client terminal are not limited to those configured by using articles in a real space, and may be ones configured by using articles in a virtual space, or ones configured by combining articles in the real space and articles in the virtual space. In a case of ones configured by using articles in the virtual space as described above, inputs and outputs are made in the virtual space, or a space compounded of the virtual space and the real space.

### [Configuration of each processing unit]

Next, with reference to FIGS. 2 to 7, processing units included in the estimation system S are described.

As shown in FIG. 2, the server 1 includes, as functions (processing units) implemented by at least one of a mounted hardware configuration and a program, an attribute recognition unit 10, a subject thing presentation unit 11, an affect recognition unit 12, a reference evaluation value recognition unit 13, a correlation data generation unit 14, a correlation data storage unit 15, an evaluation value estimation unit 16, and an attractiveness value estimation unit 17.

The attribute recognition unit 10 presents, to a user U via the user terminal 2, a questionnaire for confirming attributes of the user U in an answerable form, and recognizes the attributes of the user U, based on answers to the questionnaire, as shown in FIG. 3.

Here, an "attribute" of a user refers to information that directly or indirectly affects an evaluation of a thing made by the user. For example, age, gender, food allergies, physical information such as height and weight, hometown (that is, cultural background), and the like can be listed. In the estimation system S, for the attributes, age, gender, hometown, and food allergies are adopted.

Note that the attribute recognition unit 10 may be configured to recognize the attributes of a user U by referring to information acquired beforehand with regard to the user U, without referring to the questionnaire and answers thereto. Here, the information acquired beforehand is, for example, information inputted by the user U in a service rendered by another system that is provided by the provider of the service rendered by the estimation system S, or the like.

The subject thing presentation unit 11 presents, via the user terminal 2, information related to a subject thing of which an evaluation value given by the user U is to be recognized. Specifically, since the subject thing is a dish in the present embodiment, the subject thing presentation unit 11 presents an image of the dish and, in addition, information on a place where the dish can be eaten or the like.

Note that although the information related to the subject thing is presented in such a manner in the estimation system S, the method of presenting the subject thing to a user may be changed as appropriate in the present invention, as long as the method allows the user to recognize the subject thing.

Accordingly, for example, the subject thing itself (more specifically, the dish itself) may be presented to a test user, and only an image of the subject thing may be presented to a user for which an attractiveness value is to be estimated, which will be described later in the present embodiment.

However, in the evaluation value estimation system and the like of the present invention, it is preferable that the same information be presented to both a test user and a user for which an attractiveness value is to be estimated, and further, it is preferable that the information be presented under the same circumstances (for example, in terms of whether or not the subject thing itself is presented, or the like). In the evaluation value estimation system and the like of the present invention, when the subject thing is not presented on the system (for example, when only presentation of the subject thing itself is performed without presenting information, or the like), the subject thing presentation unit may be omitted.

The affect recognition unit 12 recognizes an affect of a user U at a time the user U uses the estimation system S. The affect recognition unit 12 includes an affect model presentation section 12a and a coordinate recognition section 12b (selected-coordinate recognition section).

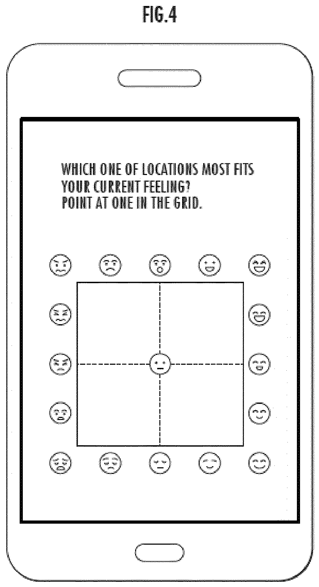

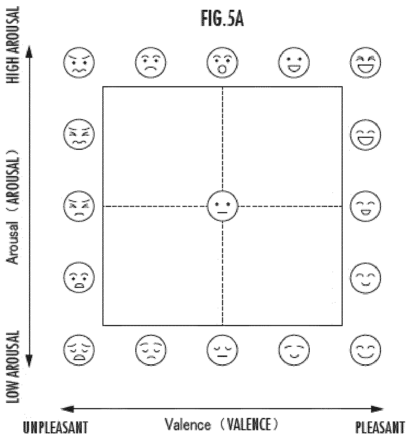

The affect model presentation section 12a presents, to the user U via the user terminal 2, an affect model that is a two-dimensional model including a first axis representing valence intensity and a second axis representing arousal, in a form in which any coordinates in the affect model is selectable, as shown in FIG. 4. In the estimation system S, for the affect model to be presented, EmojiGrid(R) shown in FIG. 5A is adopted.

Here, "valence" is a bipolar concept that indicates qualitative differences in affect evoked (for example, a positive value such as attractiveness (pleasant) of, or a negative value such as averseness (unpleasant) to, a subject thing). The "valence intensity" is the intensity of how strongly an affect of interest is felt (Harabe, Yoshihiro (2015). Measurement of general emotional valence intensity by Thurstone's method of paired comparisons. - Preliminary study: using unpleasant word - Annual report (17), Faculty of Human Sciences, Tezukayama Gakuin University).

The "arousal" is a concept that has excited (high arousal) and calm (low arousal) at polar ends, and indicates the intensity of an affect evoked (Bradley, M.M., Greenwald, M.K., Petry, M.C., and Lang, P.J. (1992). Remembering Pictures: Pleasure and Arousal in Memory. Journal of Experimental Psychology: Learning, Memory, and Cognition, 18, 379-390.).

Note that the affect model in the present invention is not limited to EmojiGrid, and may be any two-dimensional model or any three-dimensional model that includes a first axis representing valence intensity and a second axis representing arousal.

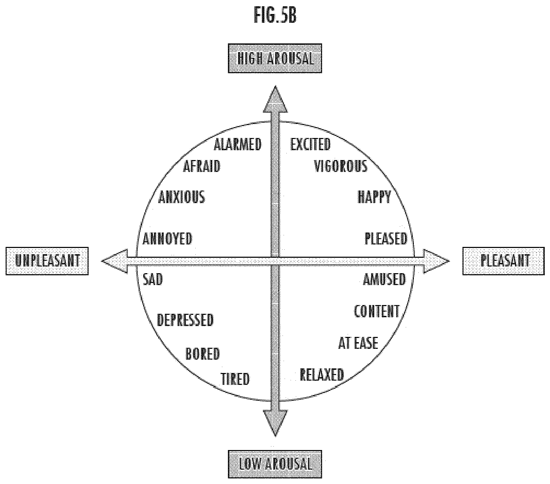

Accordingly, for example, Russell's circumplex model of affect may be adopted as the affect model, as in a modification example shown in FIG. 5B. Further, a model may be adopted that is diverted from the circumplex model of affect and in which colors or emojis are arranged in place of characters.

In the present embodiment and the modification example, cases are described in which a two-dimensional model is adopted as the affect model. However, the affect model in the present invention is not limited to such configurations, and may be a three-dimensional model created from any of the two-dimensional models and the like.

Note that for a third axis in a case where the affect model is a three-dimensional model, for example, an axis representing the degree of extent of arousal may be adopted (Sato, Toshio, Horike, Kazuya (2018). Study of emotional scale by 3-dimensional model, The 82nd Annual Convention of the Japanese Psychological Association, 721).

The coordinate recognition section 12b recognizes coordinates (selected coordinates or reference coordinates) in the affect model selected by the user U via the user terminal 2. In the estimation system S, the coordinate recognition section 12b recognizes coordinates designated by the user U touching the affect model displayed on the touch panel, which serves as the user-side input unit 20 and the user-side output unit 21 of the user terminal 2.

The coordinate recognition section 12b recognizes the recognized coordinates themselves as the affect of the user U at the time. In other words, the coordinate recognition section 12b does not particularly identify what an affect based on the coordinates is.

As described above, in the estimation system S, the affect recognition unit 12 adopts, as an interface for the user U to input an affect, the affect model that is a two-dimensional model including at least the first axis representing valence intensity and the second axis representing arousal.

Thus, in the estimation system S, the user U can easily and accurately express his/her own affect as a distinct value in the form of coordinates. As a result, it is possible to collect data related to affects of users U easily and accurately.

However, the affect recognition unit in the evaluation value estimation system and the like of the present invention is not limited to such a configuration, and may be any one that recognizes a valence intensity and an arousal experienced by a user in response to the subject thing.

Accordingly, for example, the affect recognition unit may recognize at least one of a valence intensity and an arousal as a numeric value. A method for the recognition does not necessarily need to be based on an input from a user, and the recognition may be accomplished by performing estimation or calculation based on a facial expression, an action, or the like of a user.

The reference evaluation value recognition unit 13 recognizes a reference evaluation value given to a thing that is presented when the user U uses the estimation system S.

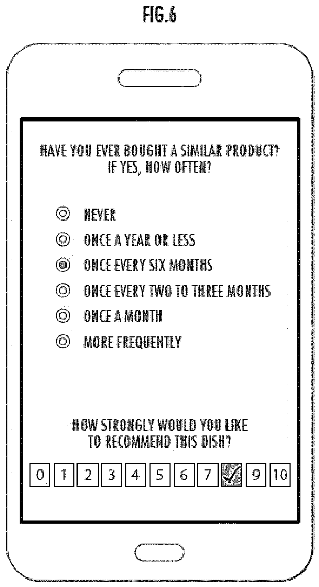

Specifically, as shown in FIG. 6, the reference evaluation value recognition unit 13 presents, to the user U via the user terminal 2, a questionnaire prepared for each predetermined evaluation metric in an answerable form, and recognizes an answer thereto as a reference evaluation value.

Here, the "evaluation metrics" are metrics for evaluation in terms of aspiration for the subject thing or a similar thing. Accordingly, "evaluation values" in such a case are values in the evaluation metrics. Note that for the evaluation metrics, adoptable are, for example, frequency of past consumption (for example, Frequency of past consumption or the like), the degree of recommendation to others (for example, Net promoter score or the like), the degree of consuming intention (for example, Willingness to take home or the like), the degree of buying intention (for example, Purchase Probability Score, Willingness to buy, the Juster scale, or the like), and the like.

The estimation system S is configured in such a manner that the user U is asked about at least one of a value concerning the frequency of past consumption, a value concerning the degree of recommendation to others, a value concerning the degree of consuming intention, and a value concerning the degree of buying intention, with regard to each of predetermined evaluation items (taste, aroma and flavor, a look, and texture in the present embodiment).

Here, the "evaluation items" are, for example, items based on five human senses in response to the subject thing. For example, when the subject thing is a dish, the taste of the dish (sense of taste), the aroma and flavor thereof (sense of smell), the look thereof (sense of vision), the texture thereof (sense of touch), the sound produced when the dish is eaten or cooked (sense of hearing), and the like are evaluation items.

The correlation data generation unit 14 generates correlation data (learning model), based on the affects (reference coordinates) of users U as test users that are recognized by the affect recognition unit 12, and the reference evaluation values given by the users U that are recognized by the reference evaluation value recognition unit 13.

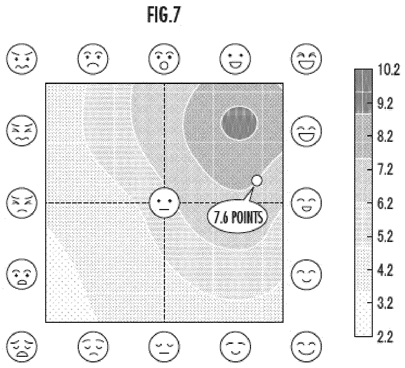

In the estimation system S, as shown in FIG. 7, the correlation data is generated as two-dimensional data that indicates correlations between coordinates selected from the affect model and specific values of one type of evaluation value. The correlation data is generated with regard to each of the plurality of evaluation metrics, with regard to each of the predetermined evaluation items (taste, aroma and flavor, a look, and texture in the present embodiment), and for each user attribute.

The correlation data thus generated is resultantly configured to receive, as an input, selected coordinates selected from the affect model by a user U as a user for which an attractiveness value is to be estimated, and output a user evaluation value.

Note that the learning model in the evaluation value estimation system and the like of the present invention is not limited to such correlation data, and may be any one that receives a valence intensity and an arousal as an input and outputs a user evaluation value.

Accordingly, for example, the learning model may be a prediction model that is generated through machine learning using, for training data, coordinates selected from the affect model and reference evaluation values that are values concerning aspiration for the subject thing or a similar thing, and that outputs an evaluation value from inputted coordinates. Moreover, an input is not limited to coordinates, and may be values of valence intensity and arousal themselves.

When processes for estimating user evaluation values that would be given to the subject thing by a user as a target audience for marketing are performed as described later, the generated learning model may be updated by using the user evaluation value estimated through a course of the process, although such a configuration is not adopted in the estimation system S.

The correlation data (learning model) in the present embodiment outputs values in the plurality of evaluation metrics with regard to each of the plurality of evaluation items, for one piece of input data (coordinates in the present embodiment, hence a valence intensity and an arousal). However, the learning model in the evaluation value estimation system and the like is not limited to such a configuration, and may be any one that receives a valence intensity and an arousal as an input and outputs a user evaluation value.

Accordingly, for example, the learning model may be one that outputs one value for one piece of input data, may be one that outputs one value for a plurality of pieces of input data, or may be one that outputs a plurality of values for a plurality of pieces of input data.

The correlation data storage unit 15 classifies the correlation data generated by the correlation data generation unit 14 by attribute of the user U and store the correlation data. Specifically, the correlation data storage unit 15 stores the correlation data in association with each attribute of the user U who inputs reference coordinates and reference evaluation values when the correlation data is generated.

The evaluation value estimation unit 16 estimates a user evaluation value that would be given by a user U to the subject thing, based on coordinates selected from the affect model and the correlation data acquired from the correlation data storage unit 15.

In the estimation system S, the evaluation value estimation unit 16 estimates, as a user evaluation value, each of a value concerning the frequency of past consumption, a value concerning the degree of recommendation to others, a value concerning the degree of consuming intention, and a value concerning the degree of buying intention, with regard to each of the predetermined evaluation items (taste, aroma and flavor, a look, and texture in the present embodiment).

This is to make multifaceted evaluations possible by recognizing values concerning the plurality of evaluation metrics, with regard to each of the plurality of evaluation items on one subject thing, and thus to make a result of analysis using such user evaluation values very objective and accurate.

However, the evaluation value estimation system and the like of the present invention are not limited to such a configuration. Accordingly, for example, the evaluation value estimation system and the like may be configured to recognize values only with regard to one evaluation item, or may be configured to recognize only values concerning one evaluation metric.

Incidentally, the evaluation value estimation unit 16 acquires from the correlation data storage unit 15 and recognizes the correlation data (learning model) corresponding to attributes of a user U, and estimates user evaluation values by using the correlation data. The reason is that when attributes of users are identical or similar, accuracy in estimation of user evaluation values is further enhanced.

However, the evaluation value estimation system and the like of the present invention are not limited to such a configuration, and may have any configuration that estimates user evaluation values by using a learning model. Accordingly, for example, the evaluation value estimation system and the like may be configured to use, when estimating user evaluation values, the same learning model even for different users, without changing learning models according to attributes of a user.

The attractiveness value estimation unit 17 estimates an attractiveness value, based on the user evaluation values estimated by the evaluation value estimation unit 16.

Specifically, first, as respective attractiveness values concerning the plurality of evaluation items (taste, aroma and flavor, a look, and texture in the present embodiment) on the subject thing (food in the present embodiment), the attractiveness value estimation unit 17 estimates values that are obtained by performing weighting based on predetermined rules (for example, attaching weights that vary from evaluation metric to evaluation metric), according to the attributes of the user U, on the respective values in the plurality of evaluation metrics (the values concerning the frequency of past consumption and the like) included in the values estimated by the evaluation value estimation unit 16 with regard to each of the plurality of evaluation items, and adding up the weighted values for each evaluation item.

Thereafter, as an attractiveness value of the subject thing itself, the attractiveness value estimation unit 17 estimates a value that is obtained by further performing weighting based on predetermined rules (for example, attaching weights that vary from evaluation item to evaluation item), according to an attribute of a user U, on the thus estimated respective attractiveness values concerning the plurality of evaluation items, and adding up the weighted values.

The reason is that there are some cases where an evaluation metric to be weighted more heavily or an evaluation item to be weighted more heavily may vary, depending on a subject thing of which user evaluation values are to be estimated, the purpose of use of user evaluation values, or the like.

Accordingly, when an attractiveness value that is a single value is outputted by adding up user evaluation values as described above, calculation of the attractiveness value is configured to be performed after attaching weights in a manner according to the purpose of use or the like. Thus, the outputted attractiveness value, and hence a result of analysis using the attractiveness value, can be made more accurate. The same applies in cases where a prediction model, not the correlation data, is used for the learning model.

However, the evaluation value estimation system and the like of the present invention are not limited to such configurations, and a method for calculating the attractiveness value may be set as appropriate.

Accordingly, for example, weighting may be performed according to the type of a subject thing, or a rule based on another metric, not according to attributes of a user as in the present embodiment, or may be performed according to a combination of a plurality of rules. A value obtaining simply by adding up user evaluation values may be used for an attractiveness value, without performing weighting. For example, weighting may be performed only with regard to a predetermined evaluation metric, or may be performed only with regard to a predetermined evaluation item.

One user evaluation value to be weighted more heavily may be determined based on the type of a subject thing, and the value may be used as it is for an attractiveness value, not a plurality of user evaluation values being added up. One or more user evaluation values may be presented as it is, without estimating an attractiveness value. In such cases, the attractiveness value estimation unit may be omitted.

Note that for a weighting rule, for example, a rule created as follows is conceivable: it is examined which evaluation item is weighted more heavily depending on at least one of attributes of a user and the type of a subject thing, and the rule is created based on a result of the examination. Moreover, for example, a rule created as follows is conceivable: it is examined which evaluation metric is weighted more heavily depending on at least one of attributes of a user and the type of a subject thing, and the rule is created based on a result of the examination. Further, a combination of such rules is conceivable.

### [Processes that are executed by each processing unit in generation of learning model]

Next, with reference to FIGS. 2 to 4, 6, and 8, a description is given of processes that are executed by each processing unit when the estimation system S generates correlation data that is a learning model.

A user U in the processes is a test user who evaluates a subject thing beforehand, for the purpose of collecting data for generating correlation data that is a learning model.

Note that in the estimation system S, the processes for generating correlation data that is a learning model are performed by presenting a subject thing itself to the user U who is a test user, as described below. However, the processes may be performed by presenting a similar thing in place of the subject thing. Further, when there is a plurality of test users, the processes may be performed by presenting the subject thing to one or some of the test users and presenting a similar thing to the others.

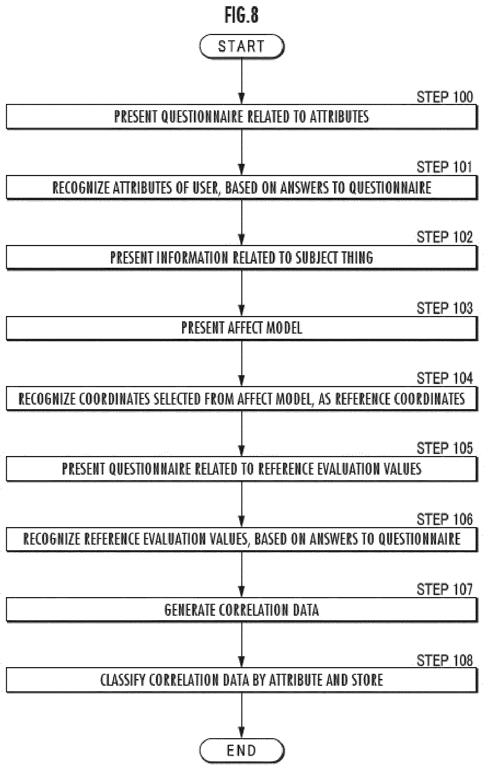

In the processes, first, the attribute recognition unit 10 presents, to the user U via the user terminal 2, a questionnaire for confirming attributes of the user U in an answerable form as shown FIG. 3 (FIG. 8, STEP 100).

Next, the attribute recognition unit 10 recognizes attributes of the user U, based on answers to the questionnaire (FIG. 8, STEP 101).

Next, the subject thing presentation unit 11 presents, to the user U via the user terminal 2, information (an image, a name, ingredients, and the like) related to a dish that is the subject thing (FIG. 8, STEP 102).

In the processes in the present embodiment, it is assumed that the dish itself is presented to the user U at the same time as the presentation of the information related to the dish that is the subject thing.

Next, the affect model presentation section 12a of the affect recognition unit 12 presents, to the user U via the user terminal 2, the affect model that is a two-dimensional model including a first axis representing valence intensity and a second axis representing arousal, in a form in which any coordinates in the affect model are selectable (FIG. 8, STEP 103).

Next, the coordinate recognition section 12b of the affect recognition unit 12 recognizes, as reference coordinates, coordinates selected by the user U via the user terminal 2 from the affect model (FIG. 8, STEP 104).

Specifically, first, the affect model presentation section 12a displays the affect model (EmojiGrid) on the touch panel of the user terminal 2 as shown in FIG. 4. Thereafter, when the user U touches any point in the affect model, the coordinate recognition section 12b determines that the coordinates of the point are selected, and recognizes the coordinates as reference coordinates.

Next, the reference evaluation value recognition unit 13 presents, to the user U via the user terminal 2, a questionnaire related to reference evaluation values to be given by the user U, in an answerable form (FIG. 8, STEP 105).

Next, the reference evaluation value recognition unit 13 recognizes reference evaluation values, based on answers to the questionnaire (FIG. 8, STEP 106).

Specifically, first, the reference evaluation value recognition unit 13 displays, on the touch panel of the user terminal 2, a plurality of questions to ask about values concerning the frequency of past consumption and the like as shown in FIG. 6. Thereafter, when the user U selects any one of answers to each question, the reference evaluation value recognition unit 13 recognizes the selected answer as a reference evaluation value in an evaluation metric corresponding to the question.

In the estimation system S, such a questionnaire by the reference evaluation value recognition unit 13 is sequentially performed with regard to each of the predetermined evaluation items (taste, aroma and flavor, a look, and texture in the present embodiment).

Next, the correlation data generation unit 14 generates correlation data (learning model), based on the affect (reference coordinates) of the user U that is recognized by the affect recognition unit 12 and the reference evaluation values given by the user U that are recognized by the reference evaluation value recognition unit 13 (FIG. 8, STEP 107).

In the estimation system S, as many pieces of such correlation data as the number of the types of reference evaluation values (that is, evaluation metrics) are generated, with regard to each of the predetermined evaluation items (taste, aroma and flavor, a look, and texture in the present embodiment).

Next, the correlation data generation unit 14 classifies the generated correlation data according to the individual attributes recognized by the attribute recognition unit 10, stores the correlation data in the correlation data storage unit 15 (FIG. 8, STEP 108), and terminates the present processes.

Note that in the present embodiment, the above-described processes are performed when information (an image, a name, ingredients, and the like) related to the dish that is the subject thing is presented to the user U who is a test user and, at the same time, the dish is eaten by the user U.

However, the processes related to generation of a learning model in the evaluation value estimation system in the present invention do not necessarily need to be performed at such a timing, and may be performed at any time before processes for estimating user evaluation values, which will be described later, are executed. Accordingly, for example, the processes related to the generation may be performed when the subject thing or a similar thing is acquired (for example, at a time of purchase or the like), apart from when the subject thing or a similar thing is presented.

The process of recognizing attributes of the user U (STEP 100 and STEP 101) may be performed at any time before the process of storing correlation data (STEP 108). Accordingly, for example, the process may be performed beforehand independently of the series of processes, or may be performed after correlation data is generated (after STEP 107).

In the present embodiment, a case has been described in which the learning model is correlation data that indicates correlations between reference coordinates and reference evaluation values given by the user U who is a test user. However, a prediction model may be generated by performing similar processes to STEP 102 to STEP 106 and thereafter performing machine learning using, for training data, reference coordinates obtained through the processes and reference evaluation values given by a user U who is a test user, and the prediction model may be used for a learning model.

In the present embodiment, the learning model is generated by the estimation system S performing the processes as in STEP 100 to STEP 108 described above. However, the evaluation value estimation system and the like of the present invention are not limited to such a configuration, and may be configured to use a learning model that is generated separately.

### [Processes that are executed by each processing unit in estimation of user evaluation values and attractiveness value]

Next, with reference to FIGS. 2 to 4, 9, and 10, a description is given of processes that are executed by each processing unit when the estimation system S estimates user evaluation values and an attractiveness value.

A user U in the processes is a target audience for marketing of the subject thing. An attractiveness value estimated with regard to the user U is presented, via the client terminal 3, to a client that performs the marketing. Note that in the following processes, a similar thing, in place of the subject thing, may be used to perform the processes, and at least one of user evaluation values and an attractiveness value of the subject thing may be estimated by using a result of the processes.

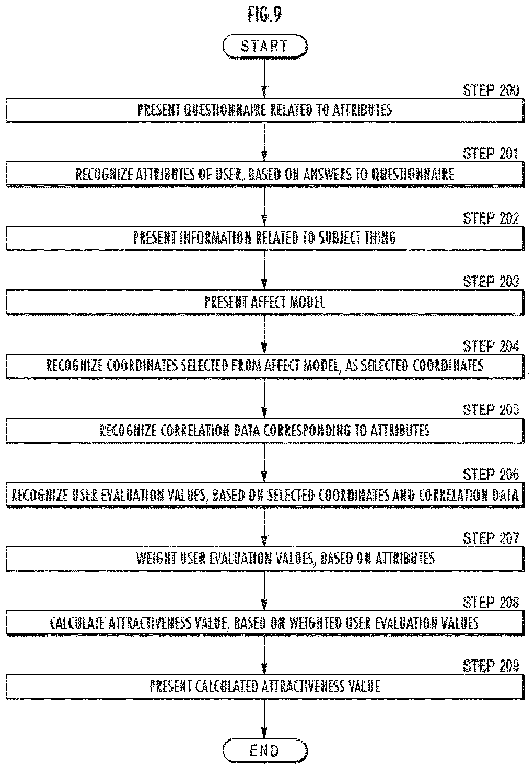

In the processes, first, the attribute recognition unit 10 presents, to the user U via the user terminal 2, a questionnaire for confirming attributes of the user U in an answerable form as shown FIG. 3 (FIG. 9, STEP 200).

Next, the attribute recognition unit 10 recognizes attributes of the user U, based on answers to the questionnaire (FIG. 9, STEP 201).

Next, the subject thing presentation unit 11 presents, to the user U via the user terminal 2, information (an image, a name, ingredients, and the like) related to a dish that is the subject thing (FIG. 9, STEP 202).

In the present process in the present embodiment, only the information related to the dish that is the subject thing is presented, and the dish itself is not presented to the user U. However, it is preferable that the presentation to the user U who is a target audience for marketing be made in the same method that is used to make presentation to a test user when correlation data (learning model) used in the present process is generated.

Next, the affect model presentation section 12a of the affect recognition unit 12 presents, to the user U via the user terminal 2, the affect model that is a two-dimensional model including a first axis representing valence intensity and a second axis representing arousal, in a form in which any coordinates in the affect model are selectable (FIG. 9, STEP 203).

Next, the coordinate recognition section 12b of the affect recognition unit 12 recognizes, as selected coordinates, coordinates selected by the user U via the user terminal 2 from the affect model (FIG. 9, STEP 204).

Specifically, first, the affect model presentation section 12a displays the affect model (EmojiGrid) on the touch panel of the user terminal 2 as shown in FIG. 4. Thereafter, when the user U touches any point in the affect model, the coordinate recognition section 12b determines that the coordinates of the point are selected, and recognizes the coordinates as selected coordinates.

Next, the evaluation value estimation unit 16 acquires, from the correlation data storage unit 15, and recognizes correlation data corresponding to the attributes recognized by the attribute recognition unit 10 (FIG. 9, STEP 205).

Specifically, from among a plurality of pieces of correlation data stored in the correlation data storage unit 15, the evaluation value estimation unit 16 acquires correlation data that is associated with at least one identical or similar attribute to any of the attributes recognized by the attribute recognition unit 10, and that has the largest number of the identical or similar attribute items, and recognizes the correlation data.

Next, the evaluation value estimation unit 16 recognizes user evaluation values, based on the recognized correlation data and the affect (selected coordinates) of the user U recognized by the affect recognition unit 12 (FIG. 9, STEP 206).

Specifically, the evaluation value estimation unit 16 uses the recognized selected coordinates as an input, and outputs, from the recognized correlation data, respective values in the plurality of evaluation metrics, with regard to each of the predetermined evaluation items (taste, aroma and flavor, a look, and texture in the present embodiment).

Next, the evaluation value estimation unit 16 performs weighting on each of the recognized user evaluation values, based on the attributes recognized by the attribute recognition unit 10 (FIG. 9, STEP 207).

Specifically, for example, the evaluation value estimation unit 16 recognizes a weighting rule preset for each attribute, and weights, according to the rules, each of the values in the plurality of evaluation metrics included in the user evaluation values.

Next, the attractiveness value estimation unit 17 calculates an attractiveness value, based on the weighted user evaluation values (FIG. 9, STEP 208).

Specifically, the attractiveness value estimation unit 17 estimates an attractiveness value for each of the evaluation items on the subject thing by adding up the values in the plurality of evaluation metrics included in the user evaluation values concerning each of the evaluation items.

Further, the attractiveness value estimation unit 17 performs weighting on the respective estimated attractiveness values for the plurality of evaluation items, according to the weighting rules acquired in STEP 207, thereafter adds up the resultant values, and thus estimates the attractiveness value for the subject thing itself.

Next, the attractiveness value estimation unit 17 presents, to the client via the client terminal 3, the calculated attractiveness value as an attractiveness value that would be given by the user U to the subject thing, as shown in FIG. 10 (FIG. 9, STEP 209), and the terminates the present processes.

As described above, in the estimation system S and the evaluation value estimation method that is executed by using the estimation system S, universal parameters, valence and arousal, are adopted. Accordingly, user evaluation values outputted as a result of such parameters being inputted are also versatile values that are not biased by cultural background or the like. In addition, valence and arousal are parameter that can be easily acquired without using special equipment.

Furthermore, since the parameters are simplified parameters, for parameters that express complex affect, analysis processing can be performed easily and quantitatively when analysis is performed by using the parameters, compared to when a conventional parameter that expresses an affect (for example, text created by a user, or the like) is used.

Accordingly, with the system and the method, it is possible to quickly and easily collect data related to affects of users U in a versatile state, and further to easily analyze the collected data.

Note that in the evaluation system S, user evaluation values are values in the plurality of evaluation metrics, with regard to each of the plurality of evaluation items. In the evaluation system S, all of such values are estimated.

However, the evaluation value estimation system and the like of the present invention are not limited to such a configuration, and at least one of the user evaluation values may be recognized (specifically, estimated, acquired, or the like) by using another system. Specifically, for example, when the subject thing is a dish, an evaluation value on taste may be estimated by the evaluation value estimation system, and an evaluation value on a look may be recognized based on actual sales.

### [Experiment examples]

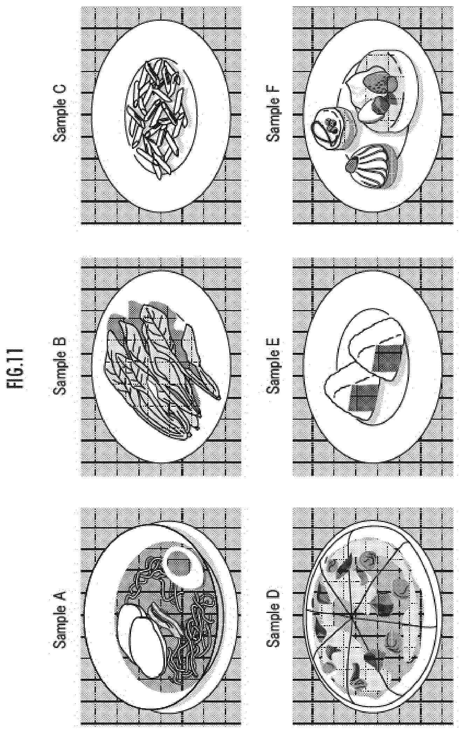

Next, with reference to FIGS. 11 to 14, a description is given of an experiment to collect actual data related to evaluation metrics and experimental data acquired through the experiment.

The experiment corresponds to STEP 100 to STEP 106 under [Processes that are executed by each processing unit in generation of learning model] described above with reference to FIGS. 7 and 8. Accordingly, experimental data acquired through the experiment is used to create correlation data that indicates correlations between reference coordinates and reference evaluation values given by a user U as a test user (see FIG. 8, STEP 107 to STEP 108), or for training data for generating a prediction model.

Individuals under the experiment are 156 men and women aged 18 and above who belong to a predetermined region (specifically, who have a nationality of a predetermined country). As to the percentages of the individuals by gender, 54.5% are men and 45.5% are women. The average age of the individuals is 40.2 years ± SD (standard deviation) of 9.5 years.

In the experiment, after six different food images (that is, similar things to a dish that is a subject thing) as shown in FIG. 11 were presented to each individual, the individual was made to input affects (valence intensity and arousal) by using EmojiGrid (see FIG. 4), and to fill out a questionnaire on the evaluation metrics (see FIG. 6). Note that FIG. 11 is an image diagram, and images in photographs of food were used in the actual experiment.

In other words, in the experiment, "a look" was adopted as an evaluation item. In the experiment, the degree of buying intention (Purchase Probability Score), the degree of consuming intention (Willingness to take home), and the degree of recommendation to others (Net Promoter Score) were adopted as evaluation metrics.

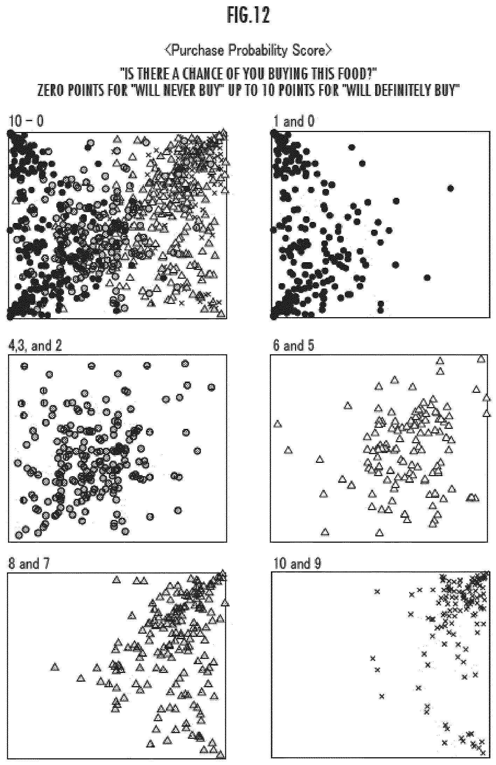

Regarding "the degree of buying intention (Purchase Probability Score)" shown in FIG. 12, the question "Is there a chance of you buying this food?" was adopted as a questionnaire, and 11-point scale values ranging from zero points representing "will never buy" to 10 points representing "will definitely buy" were adopted as evaluation values to be acquired with the questionnaire.

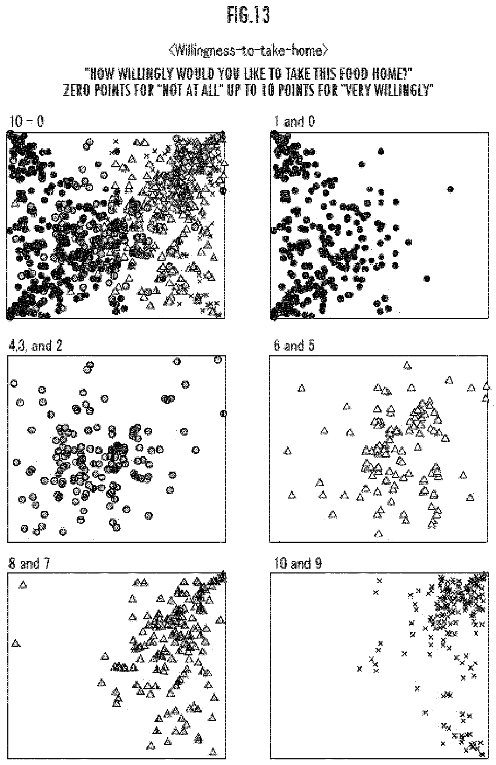

Regarding "the degree of consuming intention (Willingness to take home)" shown in FIG. 13, the question "How willingly would you like to take this food home?" was adopted as a questionnaire, and 11-point scale values ranging from zero points representing "not at all" to 10 points representing "very willingly" were adopted as evaluation values to be acquired with the questionnaire.

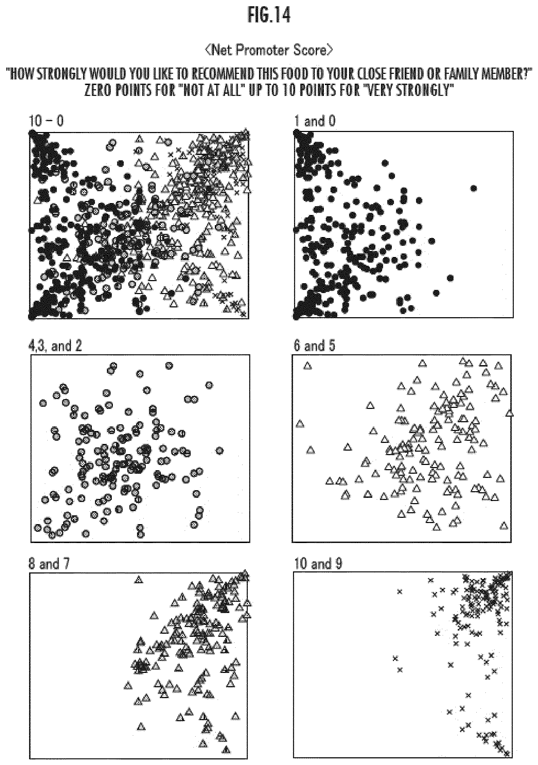

Regarding "the degree of recommendation to others (Net Promoter Score)" shown in FIG. 14, the question "How strongly would you like to recommend this food to your close friend or family member?" was adopted as a questionnaire, and 11-point scale values ranging from zero points representing "not at all" to 10 points representing "very strongly" were adopted as evaluation values to be acquired with the questionnaire.

FIGS. 12 to 14 are experimental data collected. Of the data in each figure, upper-left data is a result of plotting inputted affects (coordinates selected in EmojiGrid) with regard to all evaluation values. Upper-right data is a result of plotting affects inputted when evaluation values are zero and one. Middle-left data is a result of plotting affects inputted when evaluation values are two, three, and four. Middle-right data is a result of plotting affects inputted when evaluation values are five and six. Lower-left data is a result of plotting affects inputted when evaluation values are seven and eight. Lower-right data is a result of plotting affects inputted when evaluation values are nine and ten.

As shown in FIGS. 12 to 14, it has been found that there is a clearly significant correlative relationship between evaluation values and affects. Accordingly, an idea has been conceived that a significant learning model can be generated if such data is used.

Here, as described above, the estimation system S adopts, as an interface for a user U to input an affect, the affect model (EmojiGrid) that is a two-dimensional model including at least a first axis representing valence intensity and a second axis representing arousal, with which a user U can easily and accurately express his/her own affect as a distinct value in the form of coordinates.

Since the parameters in the form of coordinates are parameters that can be intuitively understood by a user U, it is not necessary to give users a particular explanation about a method for expression when collecting data. Thus, in the estimation system S, it is possible to quickly collect parameter values related to an affect that can easily change before the affect changes.

Since the parameters in the form of coordinates are simple and distinct, analysis processing can be performed more easily if such coordinates are used for the parameters to perform analysis.

### [Other embodiments]

Although the embodiment shown in the drawings has been described hereinabove, the present invention is not limited to the illustrated forms.

For example, in the embodiment, a case is described in which the estimation system S is a single computer system. However, apart from such a computer system, the present invention also includes an evaluation value estimation program for causing any one or more computers to execute the evaluation value estimation method, and a computer-readable recording medium on which the program is recorded and from which a user uses the program.

In the embodiment, an attractiveness value that would be given to the subject thing by a user U as a target audience for marketing is presented to a client engaged in marketing or the like. However, the evaluation value estimation system and the like of the present invention are not limited to such a configuration.

Accordingly, for example, user evaluation values estimated by using the evaluation value estimation system and the like of the present invention, or user evaluation values and an attractiveness value, may be configured to be presented to the user of interest. At least one of the estimated user evaluation values and the estimated attractiveness value may be used directly for data for machine learning or the like in marketing or the like, not to be presented to someone.

### Reference Signs List

1 server, 2 user terminal, 3 client terminal, 10 attribute recognition unit, 11 subject thing presentation unit, 12 affect recognition unit, 12a affect model presentation section, 12b coordinate recognition section (selected-coordinate recognition section), 13 reference evaluation value recognition unit, 14 correlation data generation unit, 15 correlation data storage unit, 16 evaluation value estimation unit, 17 attractiveness value estimation unit, 20 user-side input unit, 21 user-side output unit, 30 client-side output unit, S estimation system (evaluation value estimation system), U user.

## Claims

1. An evaluation value estimation system that estimates a user evaluation value that is a value concerning aspiration of a user for a subject thing, the evaluation value estimation system comprising:
an affect recognition unit that recognizes valence intensity and arousal of the user toward the subject thing; and
an evaluation value estimation unit that estimates the user evaluation value by using a learning model that receives the valence intensity and the arousal of the user as an input and outputs the user evaluation value.

2. The evaluation value estimation system according to claim 1, wherein
the affect recognition unit includes: an affect model presentation section that presents, to the user, an affect model that is a two-dimensional model or a three-dimensional model including at least a first axis representing valence intensity and a second axis representing arousal, in a form in which any coordinates in the affect model are selectable; and a selected-coordinate recognition section that recognizes selected coordinates that are coordinates selected by the user from the affect model when the subject thing is presented, and
the evaluation value estimation unit receives the selected coordinates as an input.

3. The evaluation value estimation system according to claim 1, wherein
the user evaluation value includes values concerning a plurality of evaluation items, and
the learning model receives one set of values of the valence intensity and the arousal as an input and outputs the values concerning the plurality of evaluation items.

4. The evaluation value estimation system according to claim 3, comprising an attractiveness value estimation unit that estimates, as an attractiveness value, a value obtained by weighting each of the values concerning the plurality of evaluation items outputted from the learning model as a result of the one set of values of the valence intensity and the arousal being inputted, and adding up the weighted values.

5. The evaluation value estimation system according to claim 3, wherein
the subject thing is food, and
the plurality of evaluation items includes at least one of an evaluation item based on a sense of taste, an evaluation item based on a sense of smell, an evaluation item based on a sense of vision, and an evaluation item based on a sense of touch.

6. The evaluation value estimation system according to claim 1, wherein
the user evaluation value includes values concerning a plurality of evaluation metrics, and
the learning model receives one set of values of the valence intensity and the arousal as an input and outputs the values based on the plurality of evaluation metrics.

7. The evaluation value estimation system according to claim 6, comprising an attractiveness value estimation unit that recognizes, as an attractiveness value, a value obtained by weighting each of the values concerning the plurality of evaluation metrics outputted from the learning model as a result of the one set of values of the valence intensity and the arousal being inputted, and adding up the weighted values.

8. The evaluation value estimation system according to claim 6, wherein the values concerning the plurality of evaluation metrics include at least one of a value concerning frequency of past consumption, a value concerning a degree of recommendation to others, a value concerning a degree of consuming intention, and a value concerning a degree of buying intention.

9. The evaluation value estimation system according to claim 2, wherein the learning model is correlation data indicating a correlation between reference coordinates and a reference evaluation value, the reference coordinates being coordinates selected from the affect model, the reference evaluation value being a value concerning aspiration for the subject thing or a similar thing having an identical or similar attribute to an attribute of the subject thing.

10. The evaluation value estimation system according to claim 9, wherein
the reference coordinates are coordinates selected with regard to the subject thing or the similar thing from the affect model by a test user who evaluates the subject thing or the similar thing beforehand, and
the correlation data is data indicating a correlation between the reference coordinates and the reference evaluation value given by the test user.

11. The evaluation value estimation system according to claim 2, wherein the learning model is a prediction model that is generated through machine learning using reference coordinates and a reference evaluation value for training data, and that outputs the user evaluation value from the selected coordinates inputted, the reference coordinates being coordinates selected from the affect model, the reference evaluation value being a value concerning aspiration for the subject thing or a similar thing having an identical or similar attribute to the subject thing.

12. The evaluation value estimation system according to claim 11, wherein
the reference coordinates are coordinates selected with regard to the subject thing or the similar thing from the affect model by a test user who evaluates the subject thing or the similar thing beforehand, and
the prediction model is generated through machine learning using the reference coordinates and the reference evaluation value given by the test user for training data.

13. An evaluation value estimation device that estimates a user evaluation value that is a value concerning aspiration of a user for a subject thing, the evaluation value estimation device comprising:
an affect recognition unit that recognizes valence intensity and arousal of the user toward the subject thing; and
an evaluation value estimation unit that estimates the user evaluation value by using a learning model that receives the valence intensity and the arousal of the user as an input and outputs the user evaluation value.

14. An evaluation value estimation method of estimating a user evaluation value that is a value concerning aspiration of a user for a subject thing, the evaluation value estimation method comprising:
a step of, by an affect recognition unit, recognizing valence intensity and arousal of the user toward the subject thing; and
a step of, by an evaluation value estimation unit, estimating the user evaluation value by using a learning model that receives the valence intensity and the arousal of the user as an input and outputs the user evaluation value.

15. An evaluation value estimation program that causes a computer to execute an evaluation value estimation method of estimating a user evaluation value that is a value concerning aspiration of a user for a subject thing, the evaluation value estimation program causing the computer to execute:
a step of, by an affect recognition unit, recognizing valence intensity and arousal of the user toward the subject thing; and
a step of, an evaluation value estimation unit, estimating the user evaluation value by using a learning model that receives the valence intensity and the arousal of the user as an input and outputs the user evaluation value.

16. A recording medium on which the evaluation value estimation program according to claim 15 is recorded, and from which the evaluation value estimation program is readable by the computer.
